# EUROPEAN PATENT SPECIFICATION

(11) **EP 0 294 703 B1**
(45) Date of publication and mention of the grant of the patent: **10.05.1995**
(21) Application number: 88108793.6
(22) Date of filing: 01.06.1988
(51) Int. Cl.: A61K 39/44, A61K 38/22, A61K 45/00, A61K 45/05

(54) **Bifunctional antibody constructs and method for selectively destroying cell populations**
Bifunktionelle Antikörperkonstruktionen und Verfahren zur selektiven Tötung von Zellbeständen
Construction bifonctionnelle d'anticorps et procédé de destruction sélective de populations de cellules

(30) Priority: 10.06.1987 US 60205
(43) Date of publication of application: 14.12.1988
(73) Proprietor: DANA-FARBER CANCER INSTITUTE, INC., Boston, MA 02115 (US)
(72) Inventor: Schlossman, Stuart F., Newton Center, MA 02159 (US); Scott, Charles F., Jr., Boston, MA 02155 (US); Lambert, John M., Brookline, MA 02146 (US); Blattler, Walter A,, Brookline, Ma. 02146 (US)
(74) Representative: Hansen, Bernd, Dr. Dipl.-Chem.

(56) References cited:
- WO-A-88/03565
- WO-A-88/08716
- US-A- 4 676 980
- MOLECULAR IMMONULOGY, vol. 26, 1989; SWACK et al., pp. 1037-1049#
- JOURNAL OF IMMUNOLOGY, vol. 143, 1989; GANGEMI et al., pp. 2439-2447#
- JOURNAL OF IMMUNOLOGY, vol. 140, 1988; MORIMOTO et al., pp. 2165-2170#
- DICT. IMMUNOLOGY, 3rd ed., Blackwell Science Publ., W.J. Herbert (Ed.); pp. 2-3#
- PROCEEDINGS OF THE NATL. ACADEMY OF SCIENCES USA, vol. 82, December 1985, Washington, DC (US); M.A. LIU et al., pp. 8648-8652#
- CELL, vol. 36, 1984, Cambridge, MA (US); S.C. MEUER et al., pp. 897-906#
- JOURNAL OF IMMUNOLOGY, vol. 140, no. 1, 01 January 1988, American Association of Immunologists, US; C.F. SCOTT jr. et al., pp. 8-14#
- JOURNAL OF IMMUNOLGY, vol. 135, no. 1, July 1985, Baltimore, MD (US); R.E. SCHMIDT et al., pp. 672-678#

## Description

### FIELD OF THE INVENTION

The present invention relates to novel antibody constructs and to their use for selectively destroying cell populations. In particular, the present invention relates to novel bifunctional antibody constructs selected from heterodimeric antibody conjugates (sometimes referred to herein as "antibody heterodimers"), which have dual binding specificity to both activated lymphocytes and cell surface molecules, and to the use for destroying specific target cells, including tumor cells or other diseased cells by recruiting activated lymphocytes such as activated T cells and natural killer cells to attack the target cells selectively by means of the novel antibody constructs.

### BACKGROUND OF THE INVENTION

The use of activated populations of lymphoid cells to lyse or kill tumor targets in the therapy of neoplasia has recently been a subject of intense research interest (Staerz W.D. and Bevan M.J., Immunol. Today 7:241-245 (1986)). Specifically, populations of peripheral lymphocytes, activated by the T-cell derived glycoprotein, interleukin-2 (IL-2), that is, lymphokine activated killer cells (LAK cells), have been used to successfully treat tumors in vivo in animal models (Mazumler A. and Rosenberg S.A., J. Exp. Med. 159:495-507 (1984)), and recently have been used for the treatment of human tumors resistant to standard therapeutic regimens (Rosenberg S.A. et al., NEJM 316:889-897 (1987)). This form of treatment has been limited, however, by the toxicity of IL-2, which must be given in extremely large doses to be effective (Rosenberg S.A., et al., NEJM 316:889-897 (1987)). Thus, while the concept of using activated lymphoid cells to treat malignancy remains of great interest, attention has turned to potentially less toxic means of in vitro and in vivo activation of these cells.

Lymphoid cells express a variety of lineage-specific surface antigens, some of which are directly involved in the activation of these cells to a cytolytic function (Reiherz E.L., Meuer S.C. and Schlossman S.F., Immunol. Rev. 74:83-112 (1983)). So far, two major surface structures on lymphoid cells have been shown to be involved in this cytolytic activation, namely, the T-cell antigen-receptor/T3 complex (TCR-T3) and the T11 molecule, also known as the E-rosette receptor (Meuer S.C., et al., Cell 36:897-906 (1984) and Oettgen H.C. and Terhorst C., Human Immunol. 18:187-204 (1987)). TCR-T3 is expressed on all mature cytolytic T cells (CTL), and on a few "natural killer" cells (NK cells), and T11 is expressed on both CTL and NK cells (Reinherz E.L., et al., Immunol. Rev. 74:83-112 (1986); Schmidt R., et al., J. Exp. Med. 164:351-356 (1986)). More specifically, certain antibodies to the TCR-T3 complex, or to T11, have been shown to induce and/or enhance killing by CTL or NK cells (Spits H., et al., Eur. J. Immunol. 15:85-91 (1985); Siliciano R.F. et al., Nature 317:428-429 (1985)). Furthermore, it has recently been found that human CTL can be induced to kill irrelevant human tumor targets (i.e., any human target, not just those cells to which the CTL are normally specific) by incubating these CTL and targets in vitro in the presence of bifunctional hybrid antibodies or heterodimeric antibody conjugates which recognize T3 or the T cell receptor (TCR) and a surface determinant on the tumor (Perez P., et al., Nature 316:354-356 (1985); Staerz et al., Nature 314:628-631 (1985) and PNAS 83: 1453-57 (1986); Liu et al PNAS 82:8648-8652 (1985)). Presumably, the hybrid antibody or heterodimeric antibody conjugate allows close cell-cell contact between the T cell and the tumor target by a bridging effect of the bifunctional hybrid antibody or heterodimeric antibody conjugate, with one end of the hybrid antibody or one antibody of the heterodimeric antibody conjugate binding to the tumor cells, and the other end of the hybrid antibody or the other antibody of the heterodimeric antibody conjugate binding to the TCR-T3 complex and activating the CTL. While this approach shows great promise in vitro, the in vivo infusion of such hybrid antibodies or heterodimeric antibody conjugates might be complicated by the fact that the enormous amount of non-activated, normal T cells in the body express T3. These cells would compete with activated T cells for binding to the hybrid antibody or heterodimeric antibody conjugate. Furthermore, the anti-T3/tumor antibody hybrid and conjugates would not activate the majority of NK cells, which are T3 negative.

WO-A-88/08716 describes an antigen called 2H1 and antibodies thereto, called anti-2H1. Further, those antibodies are able to induce T-cell activation or proliferation binding to non-activated T cells in synergy with a second signal.

Liu et al, Proc. Natl. Acad. Sci U.S.A. 82, 8648 (1985) describe heteroantibodies wherein one of the monoclonal antibodies is directed to T3, an antigen found on all T cells (activated or non-activated). Those heteroantibodies plus T cells are capable of killing target cells to which the second monoclonal antibody binds.

Meuer et al., Cell 36, 897 (1984) describe the T11 system as being an alternative pathway of T cell activation by, demonstrating T cell proliferation on binding of anti-T11₂ and anti-T11₃ antibodies to T cells.

### SUMMARY OF THE INVENTION

Accordingly, one object of the present invention is to provide antibody constructs for selectively destroying cell populations when using these antibody constructs wherein non-activated normal T cells in the body will not compete with activated T cells for binding to the antibody constructs.

Another object of the present invention is the use of these antibody constructs for selectively destroying cell populations wherein a majority of both the CTL cells and NK cells are activated.

Subject matter of the present invention is a bifunctional heterodimeric antibody conjugate which comprises (a) an anti-target cell antibody joined to (b) an anti-lymphocyte antibody component wherein the anti-lymphocyte antibody is specific for activated lymphocytes and does not bind to normal non-activated lymphocytes acccording to Claim 1.

Embodiments of this antibody conjugate are subject matter of Claims 2, 3 and 4.

Further subject matter is the use of a bifunctional heterodimeric antibody conjugate according to the present invention as claimed in Claims 5 to 12.

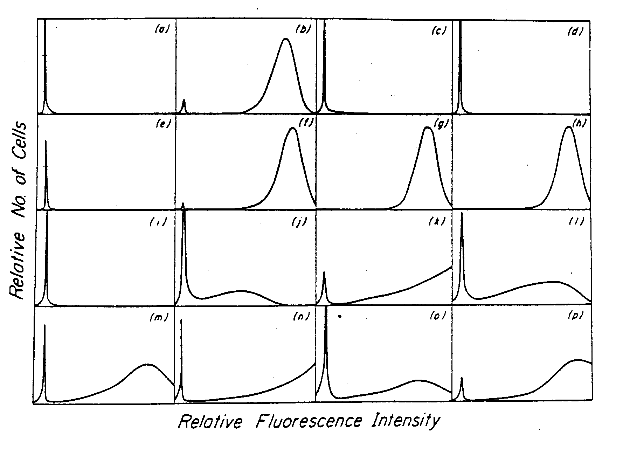

### BRIEF DESCRIPTION OF THE FIGURE

The Figure gives Fluorescence-Activated Cell Sorter (FACS) patterns showing the binding of J5, anti-T11₂, anti-T11₃, and conjugates J5-anti-T11₂ and J5-anti-T11₃ to CALLA-expressing and T11₂/T11₃-expressing cells: Namalwa cells (groups a through h) or REX cells (groups i through p) incubated with no antibody (a,i), J5 (b,j), anti-T11₂ (c,k), anti-T11₃ (d,l), anti-T3 (e,m), J5-anti-T11₂ (f,n), J5-anti-T11₃ (g,o) or J5-anti-T3 (h,p).

### DETAILED DESCRIPTION OF THE INVENTION

As noted above, the present invention provides an "alternative activation" pathway using the T11 molecule, presenting several advantages over the above described prior TCR-T3 pathway of activation. First, the T11 molecules are present on all NK cells as well as CTL cells, and the appropriate anti-T11 antibodies or T11 binding site as part of a heterodimeric antibody conjugate can recruit both populations of lymphoid cells to lyse tumor targets. Secondly, the epitope of the T11 molecule whose expression is required for activation is not expressed in the resting, non-activated state, unlike TCR-T3. This so-called "T11₃" epitope is only expressed on T11-positive cells after activation with mitogen, antigen, lymphokines, or other antibodies, such as anti-T11₂ (Meuer S.C., et al., Cell 36:897-906 (1984)), anti-T3 and anti-2H1.

The novel bifunctional antibody constructs according to the present invention, can be used to direct only activated lymphocytes toward target cells such as tumor cells. This is achieved by binding, for example, tumor cells, in vitro or in vivo, to lymphocyte-specific/tumor cell-specific bifunctional antibody constructs wherein the lymphocyte specific component is an antibody specific for activated lymphocytes. Such antibodies include, for example, the anti-T11₃ antibody, the anti-TAC antibodies that are specific for the IL2 receptor, the anti-Ta₁ antibody, the anti-T10 antibody that is specific for the T10 molecule and anti-transferrin receptor antibodies specific for the transferrin receptor (Meuer, S.C. et al., Cell 36:897-906 (1984); Fox, D.N. et al., J. Imm. 133:1250-1256 (1984); Hercendt, T. et al., Hum. Immunol. 3:247-259 (1981); and Robb, R.J. et al., J. Exp. Med. 160:1126-1146 (1984)). In this approach, the lymphocyte-specific/target cell-specific antibody construct will bind to the target tumor cells in vitro or in vivo after infusion into the body. After the antibody construct has bound to the target cells, lymphocytes can be activated in vivo or ex vivo in a variety of ways, but preferably by treatment of the lymphocytes with the combination of antibodies anti-T11₂ and anti-T11₃ which activate both T cells and NK cells. These activated lymphocytes then "home-in" on the target cells coated with the antibody construct having an anti-lymphocyte antibody specific for the activated lymphocytes, bind to the antibody construct which brings the lymphocytes in contact with the target cells, and the bound activated lymphocytes lyse the target cells.

In this application, the production of a number of antibody constructs of anti-T11₃ with antitumor antibodies is disclosed. It has now been discovered that these constructs cause activated T11-expressing cells to kill tumor target cells. Furthermore, these constructs activate T11-expressing cells to kill tumor target cells when the T11-expressing cells can also bind anti-T11₂. Also, the production of constructs of anti-T11₂ with antitumor antibodies is disclosed. It has been discovered that these antibody constructs activate T11-expressing cells to kill tumor target cells when the T11-expressing cells also bind anti-T11₃.

### The Bifunctional Antibody Constructs And Their Synthesis

The bifunctional antibody constructs provided by the present invention are heterodimeric antibody conjugates.

The term "bifunctional" is used to indicate that the constructs have two different binding specificities. The term does not relate to the actual number of binding sites since, for example, a heterodimeric antibody conjugate would normally have four binding sites -- two specific for the target cells and two specific for the activated lymphocytes.

More specifically, according to the present invention, the bifunctional heterodimeric antibody conjugates comprise two antibodies joined together, one antibody being specific to the target cells and the other antibody being specific to activated lymphocytes.

The term "target cell" as used herein is defined as any cell, including tumor cells or other diseased cells e.g. virus infected cells, parasite infected cells, etc., which are to be killed or lysed,

Specific examples of target cells which can be employed include tumor cells, especially solid tumor cells, virus infected cells, parasite infected cells, autoimmune cells, i.e. cells which produce autoantibodies, and activated cells, i.e. those which are involved in graft rejection or graft vs. host disease.

The term "lymphocyte" as used herein is defined as a mononuclear cell which circulates in the blood and is found in lymph nodes, spleen and the lymphatics, which has a nucleus that has densely packed chromatin and a small rim of cytoplasm on staining with Wright's stain, and which has potential for cytolytic function, i.e. can lyse target cells.

Examples of suitable lymphocytes include CTL (CD8+ or CD4+), NK cells (CD2+), and T helper cells (CD4+).

Also, the term "activated lymphocyte" as used herein means a lymphocyte whose cytolytic function has been turned on by various means, some of which are described below.

The antibodies specific to the target cells can be either polyclonal or monoclonal, and are of any isotype: IgM, IgG, IgG₂ₐ, IgG_{2b}, IgG₃, IgG₄, IgA, IgE and IgD. Further the antibodies should be of human, mouse, rat, hamster or any other mammalian species origin.

The antibodies specific to the target cells can be produced and purified according to conventional methods as follows.

First, mice, rats, hamsters, or any other mammal, including humans are immunized with the antigen of interest, such as the intact target cell, antigens isolated from the target cell, whole virus, attenuated whole virus and viral proteins such as viral coat proteins. In most cases, the entire target cell is used for immunization. The antigens used for immunization are isolated and purified by conventional techniques, see, e.g., R.I. Mishell in "Selected Methods in Cellular Immunology" (B.B. Mishell & S.M. Shiigi, eds.) San Francisco, (1980) pp. 28-65. Alternatively, pre-immune humans are identified by a serologic screen. Those who are serologically demonstrated to produce the antibodies of interest, e.g., anti-lymphocyte antibodies, such as patients with systemic lupus erythematosus or other autoimmune diseases or those who produce anti-tumor antibodies are selected.

Next, a source of B-cells (spleen, peripheral blood, lymph nodes, etc.) is taken from the immunized animals or pre-immune humans and fused with either a murine myeloma or a suitable human myeloma, such as NS-1 (P3/NS1/1-Ag4-1) (Kohler, G. et al., Eur. J. Imm. 6:292-295 (1976) -- (ATCC No. TIB 18)) or SP2/0 (SP2/O-Ag14) (M. Shulman et al., Nature 276:269-270 (1978) -- (ATCC No. CRL 1581)) by conventional techniques such as, for example, the polyethylene glycol (PEG) technique, e.g., according to Oi and Hertzenberg in "Selected Methods in Cellular Immunology" (Mishell, B.B. & Shiigi S.M. eds.) San Francisco, (1980) pp. 351-368. The fused cells are then cultured, screened, and cloned, all according to conventional techniques, such as described in, for example, Oi and Hertzenberg, mentioned above, to give an immortalized clone producing the antibody of choice.

The conventional technique of in vitro immunization can also be used (R.A. Luben et al., Mol. Immunol. 17:635-639 (1980) and Hengartner H. et al., Curr. Top. Microbiol. Immunol. 81:92-99 (1978).

After isolating the clones of interest, the monoclonal antibodies can be produced by hybridoma cells grown as ascites tumors in Balb/c or nude mice, or grown in vitro by using conventional large scale cell culture techniques. Antibodies can be purified from ascites fluid using a variety of methods including ammonium sulfate precipitation, ion-exchange chromatography and gel filtration (Parham et al., J. Immunol. Methods 53:133-173 (1982)), ion-exchange chromatography on resins coupled to Cibacron blue dye (Bruck et al, J. Immunol. Methods 53:313-319 (1982) and Bruck et al., Methods in Enzymology 121:587-596 (1986)), chromatography on columns of hydroxylapatite (Stanker et al., J. Immunol. Methods 76:157-169 (1985) and Juarez-Salinas et al., Methods in Enzymol. 121:615-622 (1986)) and affinity chromatography using immobilized protein A from Staphylococcus aureus (Goding, J. Immunol. Methods 39:285-308 (1980)). The purity of the antibody can be estimated by isoelectrofocusing gel electrophoresis (Goldmacher et al., J. Immunol. 136:320-325 (1986)).

Specific examples of antibodies that bind to target cells are described in Example 1 below.

Production and purification of polyclonal antibodies specific to the target cells can be carried out according to conventional techniques known in the art.

The antibodies specific to the activated lymphocytes can be either polyclonal or monoclonal, and are of any isotype as described above for the antibodies specific to the target cells. Further, as for the antibodies specific to the target cells, the antibodies specific to the activated lymphocytes should be of mammalian species origin.

The antibodies specific to the activated lymphocytes can be produced and purified as follows.

The antibodies are produced by immunizing mice with human lymphoid cells and by producing immortalized antibody producing clones as described above for producing antibodies to target cells. The human lymphoid cells are obtained from the peripheral blood of any suitable human donor. Clones of interest are identified by, e.g., FACS (Fluorescent-Activated Cell Sorter) analysis of supernatants of clones on relevant cells. The clones are then grown in the appropriate mouse strain and large quantities of the antibodies can be produced from hybridoma cells grown as malignant ascites tumors. The antibody can also be produced in vitro in large quantities by standard methods, such as growing the hybridoma cell in hollow-fiber apparatus or roller bottles. These are well-known commercial processes and the necessary equipment can be obtained commercially along with appropriate instructions for use.

The monoclonal antibodies can be purified by the methods described above.

Specific examples of two such antibodies, i.e. anti-T11₃ and anti-T11₂ are described in Example 1 below.

Production and purification of polyclonal antibodies specific to the activated lymphocytes can be carried out according to conventional techniques known in the art.

Further, several suitable antibodies specific to activated lymphocytes already exist and are publicly available including the hybridomas that produce anti-T11₂ (murine IgG₂ₐ) and anti-T11₃ (murine IgG₃) which are monoclonal antibodies that bind to different epitopes of the T11 molecule found on the surface of mature T cells (Meuer, S.C., et al., Cell 36:897-906 (1984)); anti-transferrin receptor antibody; and anti-TAC antibody. Anti-T11₂ and anti-T11₃ are commercially available from ImmunoGen Inc., Cambridge, MA. Anti transferrin receptor antibody is available from the American Type Culture Collection, Rockville, MD (ATCC No. HB21). A hybridoma that produces anti-TAC antibodies is commercially available from Becton Dickinson, Mountain View, CA.

Anti-T11₃ is preferred as an anti-lymphocyte antibody.

These publically available antibodies can be produced (if supplied by means of a hybridoma) and purified (if necessary) by the methods described above.

In order to prepare the heterodimeric antibody conjugates of the present invention, the two different antibodies may be joined together by using any of the conventional procedures for chemical cross-linking of proteins such as described by Peters et al., Annual Review Biochem. 46:523-551 (1977); Ji. Methods Enzymol. 91:580-609 (1983); Blattler et al., Biochemistry 24:1517-1524 (1985), and references therein.

Many of these cross-linking reagents are available commercially, for example from Pierce Chemical Company, Rockford, IL, Sigma Chem. Co., St. Louis, MO, Boehringer Mannheim Biochemicals, Indianapolis, IN and Pharmacia, Uppsala, Sweden. Preferably, the two different antibodies are modified with heterobifunctional cross-linking reagents which will introduce complimentary reactive groups, for example maleimido groups and sulfhydryl groups, and then the different modified antibodies are subsequently cross-linked by a reaction of the complimentary groups that yields only the heterodimer in high yield, as illustrated in Scheme 1 below.

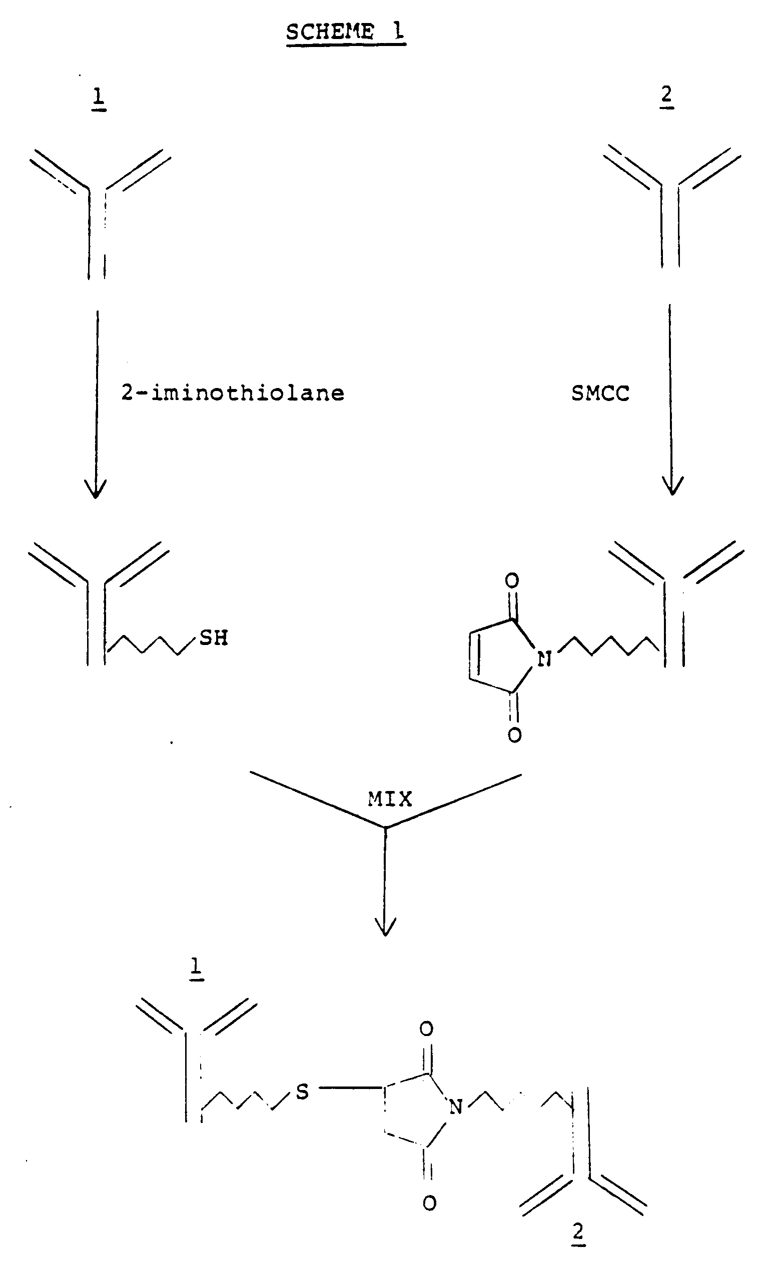

In Scheme 1, antibody 1 is modified with 2-iminothiolane which reacts with amino groups found on all proteins to introduce sulfhydryl groups into the antibody (Traut et al., Biochemistry 12:3266-3275 (1973); Lambert et al., Biochemistry 17:5406-5416 (1978)). Sulfhydryl groups may be introduced into antibodies using a number of other reagents, such as N-succinimidyl 3-(2-pyridyldithio)propionate (Carlsson et al., Biochem. J. 173: 723-737 (1978)), and by mild reduction with reducing agents by, e.g. treating antibodies with 2-mercaptoethanol (2-20 mM) or dithiothreitol (1-2 mM) for a short time (15-40 min) at 4°C or ambient temperature, followed by gel filtration or dialysis to remove excess reducing agent.

In Scheme 1, antibody 2 is modified with succinimidyl 4-(N-maleimidomethyl)cyclohexane-1-carboxylate (SMCC) which reacts with amino groups found on all proteins to introduce maleimido groups into the antibody (e.g., Lambert et al., J. Biol. Chem. 260:12035-12041 (1985)). Maleimido groups may also be introduced into proteins using other heterobifunctional reagents, for example m-maleimidobenzoyl-N-hydroxysuccinimide ester and succinimidyl 4(p-maleimidophenyl)butyrate are two compounds available commercially from Pierce Chemical Co., Rockford, IL, maleimidohexanoyl-N-hydroxysuccinimide ester is available commercially from Boehringer Mannheim Biochemicals, Indianapolis, IN, and see Blattler et al., Biochemistry 24:1517-1524 (1985). Other groups, for example α-chloro-, α-bromo-or α-iodo-carbonyl functions, are also reactive with sulfhydryl groups and such groups can be introduced into antibody 2 instead of maleimido groups to enable antibody 1 and antibody 2 to be efficiently cross-linked. For example, antibody 2 can be modified with N-succinimidyl iodoacetate to introduce iodoacetyl groups into the antibody. 2-Pyridyldithio groups are also reactive with free sulfhydryl groups and such groups can also be introduced into antibody 2 by reaction with N-succinimidyl 3-(2-pyridyldithio) propionate, for example. In this case, the cross-linking reaction between antibody 1 and antibody 2 will result in a cross-link containing a disulfide bond (see Lambert et al., J. Biol. Chem. 260: 12035-12041 (1985)).

Sulfhydryl groups can be introduced into either one of antibody 1 or antibody 2, and groups that will react with sulfhydryl groups such as maleimido groups can be introduced into either one of antibody 2 or antibody 1 for efficient formation of a cross-link between antibody 1 and antibody 2.

Sulfhydryl groups may be introduced into both antibodies, which then may be oxidized to form disulfide bonds, some of which can link antibody 1 and antibody 2. Sulfhydryl groups introduced into both antibodies may also be cross-linked by bifunctional alkyl dihalides or by bifunctional bis-maleimides such as N,N-O-phenylenedimaleimide and bismaleimidohexane.

Another method by which an antibody 1 - antibody 2 cross-link can be formed is to conjugate one of the antibodies to biotin using one of the commercially available derivatives of biotin that will react with proteins such as N-hydroxysuccinimidobiotin or sulfosuccinimidyl 6-(biotin-amido) hexanoate (Pierce Chemical Co., Rockford, IL), and to conjugate the second antibody to avidin or to streptavidin using any of the cross-linking procedures for joining two proteins that have been summarized above, or see Lambert et al., J. Biol. Chem. 260:12035-12041 (1985), for example. Avidin and streptavidin bind to biotin, and thus antibody 1 containing biotin and antibody 2 cross-linked to avidin or to streptavidin will form complexes containing both antibodies, upon mixing the two thusly-modified antibodies together.

The thus synthesized bifunctional antibody constructs are purified for further use as follows.

Heterodimeric antibody conjugates can be purified by gel filtration. Other conventional procedures can also be used including, in particular, hydroxylapatite chromatography (Juarez-Salinas et al, Meth. Enzymology 121:615-622 (1986).

Example 3 below describes in detail a method for purifying specific heterodimeric antibody conjugates. The method can be applied to the purification of any such conjugate.

The ability of the heterodimeric antibody conjugates to bind to respective cells can be evaluated as follows.

Cell lines which express on the surface antigen for one component of the heterodimer and not for the other component are used. Cells are incubated with the dimer at concentration X, for example, 50 »g/ml, or with free antibody at concentration 1/2 X, for example 25 »g/ml, for about 30 min. on ice; then the cells are washed twice and stained with an appropriate dilution of goat anti-mouse or rabbit anti-mouse immunoglobulin conjugated to a suitable label such as fluorescein isothiocyanate (FITC) for about 30 min. on ice. The cells are next washed again and assayed for fluorescence intensity on a cytofluorograph. Heterodimeric antibody conjugate, if fully functional, will give in this assay the same fluorescence intensity distribution as free antibody, thus indicating that the conjugate binds equally well to the relevant antigen on the cell.

### Activation of Lymphocytes

Lymphocytes can either be activated in vivo or ex vivo. If treatment of the target cells is in vivo and the lymphocytes are activated ex vivo, the lymphocytes are infused back into the body after activation.

In vivo activation fo lymphocytes is achieved by infusion of activating antibodies, such as anti-T11₂, anti-T3 (available from Ortho Pharmaceuticals, Raritan, NJ by the name OKT-3), or anti-2H1 several hours prior to infusion of the bifunctional antibody construct.

In vivo activation of lymphocytes can be achieved by:
(1) inducing the expression of T11₃ by infusion of anti-T11₂, anti-2H1 (an anti-2H1 producing hybridoma has been deposited with the American Type Culture Collection, Rockville, MD., Deposit No. ATCC HB 9399), or anti-T3; or
(2) inducing the expression of T11₃ as described above and activation by lymphokines, such as IL-2, BSF-1, and γ-IFN.

Preferred substances include the activating antibodies anti-T11₂, anti-T3 and anti-2H1.

Ex vivo activation of lymphocytes can be carried out for several days prior to infusion of the bifunctional antibody construct.

Ex vivo activation of lymphocytes is achieved by incubation of peripheral blood lymphocytes (PBL's) removed from blood by cytophoresis using conventional medical equipment with IL-2, anti-T11₂, anti-T3 or anti-2H1. Incubation is performed at 0.5 x 10⁶ cells/ml with soluble anti-T11₂ or anti-2H1 at, e.g., 10 »g/ml (1 »g/ml to 100 »g/ml), or IL-2 at, e.g., 10-1000 U/ml, or anti-T3 adhered to plates or bound to beads at, e.g., 10 »g/ml (1 »g/ml to 100 »g/ml) in, e.g., RPMI 1640 medium supplemented with 10% human AB serum at 37°C in an atmosphere of 100% humidity and 5-10% CO₂.

Substances that can be used to activate lymphocytes ex vivo include IL-2, anti-T11₂, anti-T3, and anti-2H1.

Preferred substances include anti-T11₂, anti-T₃, and anti-2H1, and especially preferred is anti-T11₂

The ability of substances to activate lymphocytes in vivo can be determined by infusing into the patient the activating substance, such as the antibodies mentioned above, then withdrawing blood, isolating the PBLs and assaying, e.g., for T11₃ expression and killing ability with the appropriate corresponding bifunctional antibody construct. Specific conditions of the assay can readily be determined by the skilled artisan.

The ability of substances to activate lymphocytes ex vivo can be determined by, e.g., the ability of the substances to generate killer cells after 3 days in vitro culture with the above-described activating reagents. Killer cell function is measured by monitoring the ability of activated PBL's to kill targets using one of the bifunctional antibody constructs of the present invention or by monitoring lectin mediated lysis in accordance with conventional methods.

The present invention is further illustrated by reference to a preferred embodiment but the present invention is not to be construed as being limited thereby.

In this preferred embodiment of the present invention, the heterodimeric antibody conjugate contains anti-T11₃ as the anti-lymphocyte antibody component and an anti-tumor cell antibody as the anti-target cell antibody component. According to this embodiment, the anti-T11₃/anti-tumor cell antibody conjugate can be infused prior to the infusion of activated cells without concern that the conjugate will bind to the huge number of non-activated T11-expressing cells that circulate in vivo. Thus, the very low expression of the T11₃ epitope on these circulating normal non-activated T cells allows the anti-T11₃-antitumor cell antibody conjugate to bind efficiently to the target tumor cells in vivo. When activated lymphocytes are infused subsequently, the activated lymphocytes target specifically the tumor cells that are coated with the anti-T11₃-antitumor cell antibody conjugate.

For example, in one approach, T cells can be treated ex vivo with anti-T11₂, which will expose the anti-T11₃ epitope on the surface of the T cells. Upon infusion back into the body of the host, the anti-T11₂-coated cells bind to the tumor cells coated with anti-T11₃ heterodimeric antibody conjugate. The T cells are thus activated by the binding of anti-T11₂ and anti-T11₃ and are in close cell-cell contact with the target tumor cells by the heterodimeric antibody conjugate bridge that enables the activated lymphoid cells to kill the target cells.

In Example 5, set forth below, target cells were coated with a heterodimeric antibody conjugate of anti-T11₃ and an antitumor antibody specific for the target cells. When these coated target cells were mixed with activated T11-expressing cells, the target cells and the T11-expressing cells were bound together in close cell-cell contact by the heterodimeric antibody conjugate which formed a bridge between the cells. The close cell-cell contact that results from the antibody bridge allowed the activated T11-expressing cells to lyse the target cells.

### Method of In Vivo Treatment

According to the present invention, the bifunctional antibody constructs can be delivered to the patient as an intravenous infusion in normal saline with normal human albumin added as protein carrier.

The amount of bifunctional antibody construct administered varies with age, body weight and tumor burden, but generally is in the range of 0.1 to 10 mg/kg of body weight.

Intravenous IL-2 has been shown to activate killer cells in vivo (S.A. Rosenberg at al, J. Exp. Med. 161:1169-1188 (1985)), and this method can be used to obtain activated cytolytic lymphocytes. In vivo activation of lymphocytes should also proceed as described for IL-2 for the reagents described above in the section headed "Activation of Lymphocytes".

For ex vivo activation of lymphocytes, lymphocytes are derived from the patient as described above and activated as described above. The activated lymphocytes are then returned to the body by continuous intravenous infusion at a suitable rate, such as at a rate of 10⁸ cells/hour in a volume of 50 ml/hr.

Examples of medical conditions that can be treated according to the in vivo method include malignancy of any type including, for example, cancer of the lung, breast, colon, prostate, kidney and lymphatic organs; autoimmune diseases, such as systemic lupus, rheumatoid arthritis, and multiple sclerosis; graft rejections, such as renal transplant rejection, cardiac transplant rejection, liver transplant rejection, lung transplant rejection, and bone marrow transplant rejection; graft vs. host disease; viral infections, such as CMV infection, HTLV infection, AIDS, etc.; and parasite infections, such as giardiasis, amoebiasis, shistosomiasis, etc.

### Method of In Vitro Treatment

The method of the present invention can also be performed in vitro.

Examples of medical conditions that can be treated according to the in vitro method include bone marrow transplants, autologous or allogeneic.

In vitro treatment can be carried out as follows.

PBL are harvested from the patient, activated as described above (with IL-2, or/and anti-T11₂, anti-T3 anti-2H1) for several days. Bone marrow, which contains cells to be targeted, e.g. tumor cells, are mixed with the activated cells at E/T (effector cell/target cell) ratios of 20:1 to 1:1 and the appropriate bifunctional antibody construct for 4 to 18 hours. After this time, the bone marrow cells are washed with medium containing serum and returned to the patient by i.v. infusion according to known methods.

### EXAMPLES

The present invention will now be described by reference to specific examples, which are not meant to be limiting.

Unless otherwise specified, all percents, ratios, etc. are by weight.

### Monoclonal Antibodies Described in the Examples.

The hybridoma cell lines that produce antibodies anti-T11₂, anti-T11₃, J5 and anti-B4 were produced as disclosed in the cited references.

Anti-T11₂ and anti-T11₃ are monoclonal antibodies that bind to different epitopes of the T11 molecule found on the surface of mature T cells (Meuer S.C., et al., Cell 36:897-906 (1984)).

J5 is a murine IgG₂ₐ that is specific for the Common Acute Lymphoblastic Leukemia Antigen (CALLA) (Ritz J., et al., Nature 283:583-585 (1980)).

Anti-B4 is a murine IgG₁ that is specific for the B4 antigen found on B cells and malignancies of B cell origin (Nadler L.M., et al., J. Immunol. 131:244-250 (1983)).

The hybridoma cell lines producing these antibodies were grown as ascites tumors in Balb/C mice. Alternatively, J5 and anti-B4 can be obtained commercially from Coulter Immunology, Hialeah, FL. Anti-T11₂ and anti-T11₃ antibodies can be obtained commercially from ImmunoGen Inc., Cambridge, MA.

### EXAMPLE 1

### Purification of Antibodies

All purification steps were performed at 4°C. The J5 antibody was purified from ascites fluid using conventional methods (Goldmacher et al., J. Immunol. 136:320-325 (1986); Lambert et al., J. Biol. Chem. 260:12035-12041 (1985)) using affinity chromatography on a column of protein A-Sepharose® CL-4B (Sigma Chemical Co., St. Louis, Mo.). The antibody bound by the column was eluted with 0.1 M acetic acid containing NaC1 (0.15 M). Antibody-containing fractions were immediately neutralized by the addition of one-tenth volume of 1.0 M NaHC0₃, and were then dialysed against 10 mM sodium phosphate buffer, pH 6.0, containing glycine (50 mM) and NaN₃ (0.4 mM), for further purification by ion-exchange chromatography on a column of CM-cellulose (CM-52; Whatman Chemical Separations, Clifton, N.J.) equilibrated in the same buffer. The column (30 ml bed volume for 120 mg of protein) was developed with a linear gradient of NaC1 (0 to 100 mM), and J5 eluted as a single peak. The purified antibody was finally dialysed against 10 mM potassium phosphate buffer, pH 7.2, containing NaC1 (145 mM) and then sterilized by filtration (0.22 »m Millex-GV membrane, Millipore Corp., Bedford, MA or 0.2 »m Uniflo membranes, Schleicher & Schuell, Keene, NH) and stored at a concentration of about 5 mg/ml at -70°C.

Anti-T11₂ was purified by a similar procedure, except that the CM-cellulose columns used in the second step of purification of this antibody was developed with a linear gradient of 0 to 300 mM NaC1 in the 10 mM sodium phosphate buffer, pH 6.0, containing glycine (50 mM) and NaN₃ (0.4 mM).

Anti-B4 was purified in three steps at 4°C. First, ascites fluid was diluted with 2 volumes of 10 mM potassium phosphate buffer, pH 7.2, containing NaC1 (145 mM) and then solid (NH₄)₂SO₄ was added in small portions to the continuously stirred solution over 45 min. The suspension was stirred for 1 hour. The precipitated protein was recovered by centrifugation and then dissolved (in 50 ml of protein from 50 ml of ascites fluid) in 0.1 M Tris.HCl buffer, pH 8.9, containing NaC1 (3.0 M) and dialysed against 100 vol. of the pH 8.9 buffer for 8 h. The dialysed solution was applied to an affinity column of protein A-Sepharose® CL-4B that was equilibrated in the pH 8.9 buffer, and then the column was washed with pH 8.9 buffer until the absorbance of the eluate was near zero. The anti-B4 antibody was then eluted from the column with 50 mM sodium phosphate buffer, pH 6.0, containing sodium acetate (50 mM) and NaC1 (150 mM). The antibody-containing fractions were concentrated for final purification by gel filtration through a column of Sepharose® S-300 (Pharmacia Fine Chemicals, Piscataway, NJ) equilibrated in 10 mM potassium phosphate buffer, pH 7.2, containing NaC1 (145 mM). The purified antibody was sterilized and stored as described for J5 (above).

Anti-T11₃ was purified by ammonium sulfate precipitation and affinity chromatography on protein A-Sepharose® CL-4B using the same procedure as for anti-B4, except that the antibody was eluted from the protein A-Sepharose® CL-4B column with 0.1 M acetic acid containing NaC1 (0.15 M), collecting fractions directly into tubes containing 1.0 M NaHCO₃ (one-tenth fraction volume). The antibody-containing fractions were combined and dialysed exhaustively against 40 mM potassium phosphate buffer, pH 7.2, containing NaC1 (0.58 M). The purified antibody was sterilized by filtration and stored at a concentration of about 5 mg/ml at -70°C.

### EXAMPLE 2

### Chemical Conjugation of J5 or Anti-B4 to Anti-T11₂ or Anti-T11₃

The J5 antibody (2 mg/ml) in 60 mM triethanolamine.HC1 buffer, pH 8.0, containing EDTA (1 mM) and NaC1 (100 mM) was degassed and then treated with 2-iminothiolane (0.13 mM) for 90 min at 0°C under a nitrogen atmosphere. Stock solutions of 2-iminothiolane (Sigma Chemical Co., St. Louis, MO) were prepared as described by Lambert et al., Biochemistry, 17:5406-5416 (1978). The reaction was terminated by gel filtration at 4°C through a column of Sephadex® G-25 (superfine) equilibrated with 5 mM bistris.acetate buffer, pH 5.8, containing NaC1 (50 mM) and EDTA (1 mM). Sulfhydryl groups introduced into J5 antibody in this way were quantified spectrophotometrically by the method of Ellman, Arch. Biochem. Biophys. 28:70-77 (1959): about 0.7 sulfhydryl groups per molecule of J5 were introduced by the procedure described here.

Anti-B4 antibody was modified by exactly the same procedure as J5 antibody except that the 2-iminothiolane concentration used in the 90 min incubation at 0°C was 0.26 mM.

Maleimido groups were introduced into the antibodies anti-T11₂ and anti-T11₃ using the same method for each. The antibodies (1 mg/ml) in 100 mM sodium phosphate buffer, pH 7.0, containing EDTA (1 mM) were incubated with succinimidyl 4-(N-maleimidomethyl)cyclohexane-1-carboxylate (Pierce Chemical Co., Rockford, IL) for 30 min at 30°C using a reagent concentration for each antibody that resulted in the introduction of 1.0 maleimido group per molecule: 20 »M for anti-T11₂ and 10 »M for anti-T11₃. The reagent was added from a freshly prepared 10 mM stock solution in dry dioxane. Reactions were terminated by gel filtration at 4°C through columns of Sephadex® G-25 (superfine) equilibrated with the pH 7.0 buffer. The level of incorporation of maleimido groups was measured by an assay using radiolabelled cysteine as described by Lambert et al., J. Biol. Chem. 260:12035-12041 (1985).

J5 or anti-B4 (0.5 to 1.0 mg/ml) containing introduced sulfhydryl groups, in 5 mM bistris.acetate buffer, pH 5.8, containing NaC1 (50 mM) and EDTA (1 mM), was mixed with an equal weight of an antibody (0.5 to 1.0 mg/ml of anti-T11₂ or anti-T11₃) containing introduced maleimido groups in 100 mM sodium phosphate buffer, pH 7.0, containing EDTA (1 mM). The pH of the mixture was adjusted to 7.0 by the addition of 0.5 M triethanolamine.HC1, pH 8.0, and the mixture was then incubated under nitrogen for 16 hours at 4°C. Then, 2-mercaptoethanol was added to a final concentration of 2.5 »M to block any remaining maleimido groups, and, after 30 min incubation at 4°C, iodoacetamide (2 mM) was added and incubation was continued for another 60 min in order to block all free sulfhydryl groups.

### EXAMPLE 3

### Purification of Heterodimeric Antibody Conjugates

The conjugation reaction mixture was subjected to gel filtration through a column (95 cm x 2.6 cm for 10 ml sample volume) of Bio-Gel® A-1.5 m (fine) (Bio-Rad, Richmond, CA) equilibrated in 10 mM HEPES.NaOH buffer, pH 7.6, containing NaC1 (3M). Under these conditions, antibody-antibody dimers (Mᵣ ≈ 320,000) were completely resolved from aggregates of high Mᵣ and from monomeric IgG antibodies ((Mᵣ ≈ 160,000). Samples from fractions eluting from the column were analyzed by polyacrylamide/dodecyl sulfate gel electrophoresis in gel slabs (145 mm x 90 mm x 0.75 mm) cast with acrylamide gradients (5-10% w/v). Gels were run under non-reducing conditions using the procedure of Laemmli, Nature 227:680-685 (1970); Lambert et al., J. Biol. Chem. 260:12035-12041 (1985). Fractions containing purified antibody-antibody heterodimer were pooled, concentrated to about 0.5 mg/ml using a CX-30 immersible membrane (Millipore Corp., Bedford, MA), and finally dialysed into 10 mM potassium phosphate buffer, pH 7.2, containing NaC1 (145 mM). Samples were stored frozen at -70°C.

### EXAMPLE 4

### Evaluation of Binding for the Heterodimeric Antibody Conjugates: Analysis by Fluorescence-activated Cell Sorting

The ability of the J5-anti-T11₃ and J5-anti-T11₂ conjugates to bind to CALLA-expressing and T11₂/T11₃-expressing cells was evaluated. The cell line used was Namalwa, a CALLA+, B4+, T11- Burkitt lymphoma line available from the American Type Culture Collection, Rockville, MD and having ATCC No. CRL 1432 (Int. J. Cancer 12:396-408 (1973)) and REX, a T11⁺ CALLA +/- adult T-cell leukemia cell line as an example of a T11₃ and T11₂ positive cell line. However, any such cell line or also activated peripheral blood lymphocytes from any healthy human donor can be used in place of REX.

Conjugates, or the equivalent amount of unmodified antibody (i.e., J5, anti-B4, anti-T11₂, or anti-T11₃) were incubated with 1 x 10⁶ cells in a volume of 50 »l for 30 minutes on ice, were washed twice with 1 ml of Hanks Balanced Salt Solution (HBSS) containing 1% bovine serum albumin (BSA). The cells were then incubated with a 1:25 dilution of goat-anti-mouse Ig fluorescein isothiocyanate (GAMG-FITC) in a volume of 25 »l on ice, washed twice with HBSS.1% BSA, and finally the cells were analyzed for fluorescence on a Fluorescence-Activated Cell Sorter (Epics IV). The Figure shows the patterns obtained for J5, anti-T11₂, anti-T11₃ and conjugates J5-anti-T11₂ and J5-anti-T11₃.

More specifically, the Figure shows a FACS analysis of heterodimeric conjugates wherein Namalwa cells (groups a through h) or REX cells (groups i through p) were incubated with no antibody (a,i), J5 (b,j), anti-T11₂ (c,k), anti-T11₃ (d,1), anti-T3 (e,m), J5-anti-T11₂ (f,n), J5-anti-T11₃ (g,o) or J5-anti-T3 (h,p).

It is evident from the Figure that each antibody component in the heterodimeric conjugate has retained the same binding activity and specificity as the free antibody.

### EXAMPLE 5

### Activation of Peripheral Blood Lymphocytes (PBL) by anti-T11₂/anti-T11₃ Containing Conjugates

In addition to the ability to bind cells expressing relevant antigens, and anti-T11₂/anti-T11₃ conjugates must be shown to have functional properties, i.e., must activate T11 + PBL under the appropriate circumstances. It is known that the combination of anti-T11₂ and anti-T11₃ antibodies can activate PBL to proliferate (Meuer S.C., et al., Cell 36:897-906 (1984)). Therefore, the functional integrity of the conjugates of this invention was assessed by incubating PBL in the presence of the J5-anti-T11₃ conjugate and anti-T11₂ or in the presence of J5-anti-T11₂ and anti-T11₃, and measuring proliferation of sheep erythrocyte-rosetted PBL by the incorporation of ³H-thymidine. Specifically, 5 x 10⁴ PBL were incubated for 72 hrs in flat-bottom microtitre plates in the presence of the indicated reagents as shown in Table 1 below, and were pulsed with 1 »Ci of thymidine for the last 18 hours.

The results obtained are also shown in Table 1.

**Table 1**

| Activation of Human PBL by Heterodimeric Conjugates of J5 and Anti-T11₂ or Anti-T11₃ | |
|---|---|
| In Vitro Addition | CPM ± S.D. ¹ |
| -- | 365 ± 22 |
| Con A ², 2.5 »g/cc | 42,408 ± 2262 |
| Anti-T11₂ | 730 ± 177 |
| Anti-T11₃ | 2,254 ± 1103 |
| J5-anti-T11₂ | 650 ± 390 |
| J5-anti-T11₂ + anti-T11₃ | 38,359 ± 390 |
| J5-anti-T11₃ | 408 ± 102 |
| J5-anti-T11₃ + anti-T11₂ | 30,641 ± 2,582 |
| J5 + anti-T11₂ + anti-T11₃ | 25,125 ± 545 |

| | |
|---|---|
| ¹ Counts per minute ± standard deviation | |
| ² Con A = Concanavalin A | |

It is evident from the results in Table 1 above that both the J5-anti-T11₂ and J5-anti-T11₃ conjugates contain functional T11₂ and T11₃ antibodies, respectively, as they are able to activate PBL in the presence of the appropriate free antibody.

### EXAMPLE 6

### Lysis of a CALLA+ Tumor by Human CTL in the Presence of J5-anti-T11₃ and J5-anti-T11₂ Conjugates

Clone TB1-6 (received from Dr. Chikao Morimoto), a representative example of a human CTL clone, which is specific for the EBV-transformed human B cell line, CM-EBV, was used as the effector cytolytic cell in this example. The CTL clone was incubated for 4 hours with the CALLA+ tumor Namalwa, ATCC No. CRL 1432, Int. J. Cancer 12.:396-408 (1973), which the clone does not normally lyse, or in the presence of the CALLA- tumor OVCAR available from the American Type Culture Collection. ATCC No. HTB161, Canc. Res. 44:5286-5290 (1984) in the presence of a final concentration of 10 »g/ml of J5-anti-T11₃ or J5-anti-T11₂, or other reagents as shown in Table 2 below, either 1 x 10⁵ effector cytolytic cells (TBI-6) or 0.5 x 10⁵ T cells, and 5 x 10³ ⁵¹Cr-labelled Namalwa targets were incubated in V-bottom plates in a final volume of 200 ul in medium consisting of RPMI 1640 with 10% FCS and 100 »g/ml streptomycin and 100 U/ml penicillin. After four hours, supernatants were harvested and the percent specific lysis calculated according to standard methods (Siliciano R.F., et al., Nature 317:428-429 (1985)).

The results are also shown in Table 2.

**Table 2**

| Heterodimeric Conjugates of Anti-T11₂ or Anti-T11₃ and J5 Direct the Lysis of CALLA+ Targets by a Human CTL Clone | | | |
|---|---|---|---|
| Target | In Vitro Addition | % Specific Release | |
| | | 20:1 | 10:1 |
| CM-EBV | -- | 77 | 80 |
| Namalwa | -- | 1 | 0 |
| | J5-anti-T11₃ | 6 | 8 |
| | J5-anti-T11₃ + anti-T11₂ | 36 | 41 |
| | J5-anti-T11₂ | 1 | 1 |
| | J5-anti-T11₂ + anti-T11₃ | 28 | 33 |
| | J5 + anti-T11₂ + anti-T11₃ | 18 | 16 |
| OVCAR | -- | 19 | 12 |
| | J5-anti-T11₂ + anti-T11₃ | 22 | 18 |
| | J5-anti-T11₃ + anti-T11₂ | 23 | 23 |
| | J5 + anti-T11₂ + anti-T11₃ | 27 | 21 |

It is evident from Table 2 that either the J5-anti-T11₃ or J5-anti-T11₂ conjugate, in the presence of the reciprocal anti-T11 antibody (i.e., anti-T11₂ and anti-T11₃, respectively) can direct TBI-6 to lyse the CALLA+ tumor Namalwa, but not the CALLA- tumor, OVCAR.

## Claims (Claims for the following Contracting State(s): AT, BE, CH, DE, FR, GB, IT, LI, LU, NL, SE)

1. A bifunctional heterodimeric antibody conjugate comprising (a) an anti-target cell antibody component joined to (b) an anti-lymphocyte antibody component wherein the anti-lymphocyte antibody component is specific for activated lymphocytes and does not bind to normal non-activated lymphocytes.

2. The bifunctional heterodimeric antibody conjugate of Claim 1, wherein said anti-lymphocyte antibody component (b) is an anti-T11₃ antibody.

3. The bifunctional heterodimeric antibody conjugate of Claim 1 or 2, wherein said anti-target cell antibody component (a) is one member selected from the group consisting of an anti-tumor cell antibody, an antibody to a virus infected cell, an antibody to a parasite infected cell, an antibody to an autoimmune cell, an antibody to activated cells that cause graft rejection and an antibody to activated cells that cause graft vs. host disease.

4. The bifunctional heterodimeric antibody conjugate of Claim 1, wherein said antibody components (a) and (b) of the heterodimeric antibody conjugate are joined by cross-linked complimentary reactive groups.

5. In-vitro use of a bifunctional heterodimeric antibody conjugate according to any of Claims 1 to 4, wherein the following steps are carried out:
(1) binding the bifunctional heterodimeric antibody conjugate through (a) the anti-target cell antibody component to target cells;
(2) activating lymphocytes; and
(3) binding the activated lymphocytes to (b) the anti-lymphocyte antibody component;
wherein said steps (1), (2) and (3) can be conducted simultaneously or in any order, provided that step (2) is always conducted simultaneously with or prior to step (3).

6. The use of a bifunctional heterodimeric antibody conjugate according to any of Claims 1 to 4 for the preparation of a pharmaceutical agent suitable for selectively destroying target cell populations.

7. The use of Claim 6, wherein the target cells are destroyed in vitro.

8. The use according to Claim 5, wherein said lymphocytes are activated with one member selected from the group consisting of antibodies, mitogens, antigens, and lymphokines.

9. The use of Claim 8, wherein the lymphocytes are activated with one or more antibodies.

10. The use of Claim 9, wherein the lymphocytes are activated with one or more antibodies selected from the group consisting of anti-T11₂, anti-T3, and anti-2H1.

11. The use of Claim 8, wherein the lymphocytes are activated with a mixture of anti-T11₂ and anti-T11₃ antibodies.

12. The use of Claim 8, wherein said anti-lymphocyte antibody component (b) of said bifunctional heterodimeric antibody conjugate is an anti-T11₃ antibody and said lymphocytes are activated with anti-T11₂.

## Claims (Claims for the following Contracting State(s): ES, GR)

1. A bifunctional heterodimeric antibody conjugate comprising (a) an anti-target cell antibody component joined to (b) an anti-lymphocyte antibody component wherein the anti-lymphocyte antibody component is specific for activated lymphocytes and does not bind to normal non-activated lymphocytes.

2. The bifunctional heterodimeric antibody conjugate of Claim 1, wherein said anti-lymphocyte antibody component (b) is an anti-T11₃ antibody.

3. The bifunctional heterodimeric antibody conjugate of Claim 1 or 2, wherein said anti-target cell antibody component (a) is one member selected from the group consisting of an anti-tumor cell antibody, an antibody to a virus infected cell, an antibody to a parasite infected cell, an antibody to an autoimmune cell, an antibody to activated cells that cause graft rejection and an antibody to activated cells that cause graft vs. host disease.

4. The bifunctional heterodimeric antibody conjugate of Claim 1, wherein said antibody components (a) and (b) of the heterodimeric antibody conjugate are joined by cross-linked complimentary reactive groups.

5. Process for the preparation of a bifunctional heterodimeric antibody conjugate comprising joining (a) an anti-target cell antibody component to (b) an anti-lymphocyte antibody component wherein the anti-lymphocyte antibody component is specific for activated lymphocytes and does not bind to normal non-activated lymphocytes.

6. The process of Claim 5, wherein said anti-lymphocyte antibody component (b) is an anti-T11₃ antibody.

7. The process of Claim 5 or 6, wherein said anti-target cell antibody component (a) is one member selected from the group consisting of an anti-tumor cell antibody, an antibody to a virus infected cell, an antibody to a parasite infected cell, an antibody to an autoimmune cell, an antibody to activated cells that cause graft rejection and an antibody to activated cells that cause graft vs. host disease.

8. The process of Claim 5, wherein said antibody components (a) and (b) of the heterodimeric antibody conjugate are joined by cross-linked complimentary reactive groups.

9. In-vitro use of a bifunctional heterodimeric antibody conjugate according to any of Claims 1 to 4 or prepared according to any of Claims 5 to 8 wherein the following steps are carried out:
(1) binding the bifunctional heterodimeric antibody conjugate through (a) the anti-target cell antibody component to target cells;
(2) activating lymphocytes; and
(3) binding the activated lymphocytes to (b) the anti-lymphocyte antibody component;
wherein said steps (1), (2) and (3) can be conducted simultaneously or in any order, provided that step (2) is always conducted simultaneously with or prior to step (3).

10. The use of a bifunctional heterodimeric antibody conjugate according to any of Claims 1 to 4 or as prepared according to any of Claims 5 to 8 for the preparation of a pharmaceutical agent suitable for selectively destroying target cell populations.

11. The use of Claim 10, wherein the target cells are destroyed in vitro.

12. The use according to Claim 9, wherein said lymphocytes are activated with one member selected from the group consisting of antibodies, mitogens, antigens, and lymphokines.

13. The use of Claim 12, wherein the lymphocytes are activated with one or more antibodies.

14. The use of Claim 13, wherein the lymphocytes are activated with one or more antibodies selected from the group consisting of anti-T11₂, anti-T3, and anti-2H1.

15. The use of Claim 12, wherein the lymphocytes are activated with a mixture of anti-T11₂ and anti-T11₃ antibodies.

16. The use of Claim 12, wherein said anti-lymphocyte antibody component (b) of said bifunctional heterodimeric antibody conjugate is an anti-T11₃ antibody and said lymphocytes are activated with anti-T11₂.

17. Method for in-vitro use of a bifunctional heterodimeric antibody conjugate according to any of Claims 1 to 4 or as prepared according to any of Claims 5 to 8 wherein the following steps are carried out:
(1) binding the bifunctional heterodimeric antibody conjugate through (a) the anti-target cell antibody component to target cells;
(2) activating lymphocytes; and
(3) binding the activated lymphocytes to (b) the anti-lymphocyte antibody component;
wherein said steps (1), (2) and (3) can be conducted simultaneously or in any order, provided that step (2) is always conducted simultaneously with or prior to step (3).

18. Method for the preparation of a pharmaceutical agent suitable for selectively destroying target cell populations by formulating a bifunctional heterodimeric antibody conjugate according to any of Claims 1 to 4 or as prepared according to any of Claims 5 to 8 together with a pharmaceutically acceptable carrier or diluent.

19. Method according to Claim 17, wherein said lymphocytes are activated with one member selected from the group consisting of antibodies, mitogens, antigens, and lymphokines.

20. Method of Claim 19, wherein the lymphocytes are activated with one or more antibodies.

21. Method of Claim 20, wherein the lymphocytes are activated with one or more antibodies selected from the group consisting of anti-T11₂, anti-T3, and anti-2H1.

22. Method of Claim 19, wherein the lymphocytes are activated with a mixture of anti-T11₂ and anti-T11₃ antibodies.

23. Method of Claim 19, wherein said anti-lymphocyte antibody component (b) of said bifunctional heterodimeric antibody conjugate is an anti-T11₃ antibody and said lymphocytes are activated with anti-T11₂.

## Patentansprüche (Patentansprüche für folgende(n) Vertragsstaat(en): AT, BE, CH, DE, FR, GB, IT, LI, LU, NL, SE)

1. Bifunktionales heterodimeres Ahtikörperkonjugat, umfassend (a) ein Anti-ZielzellenAntikörperkomponente, verbunden an (b) eine Antilymphocyt-Antikörperkomponente, worin die Antilymphocyt-Antikörperkomponente spezifisch für aktivierte Lymphocyten ist, und nicht an normale, nichtaktivierte Lymphocyten bindet.

2. Bifunktionales heterodimeres Antikörperkonjugat nach Anspruch 1, wordin die Antilymphocyt-Antikörperkomponente (b) ein Anti-T11₃ - Antikorper ist.

3. Bifunktionales heterodimeres Antikörperkonjugat nach Anspruch 1 oder 2, worin die Antizielzellen-Antikörperkomponente (a) ausgewählt ist aus einem Antitumorzell-Antikörper, einem Antikörper gegen eine Virus-infizierte Zelle, einem Antikörper gegen eine parasitär infizierte Zelle, einem Antikörper gegen eine Autoimmunzelle, einem Antikörper gegen aktivierte Zellen, die Transplantatabstoßung bewirken, und einem Antikörper gegen aktivierte Zellen, die Transplantat/Wirtskrankheit verursachen.

4. Bifunktionales heterodimeres Antikörperkonjugat nach Anspruch 1, worin die Antikörperkomponenten (a) und (b) des heterodimeren Antikörperkonjugats durch Vernetzung komplimentär reaktiver Gruppen verbunden sind.

5. **In vitro** Verwendung eines heterodimeren Antikörperkonjugats nach einem der Ansprüche 1 bis 4, worin die folgenden Schritte durchgeführt werden:
(1) Binden des bifunktionalen heterodimeren Antikörperkonjugats durch (a) die Antizielzellen-Antikörperkomponente an die Zielzellen;
(2) Aktivieren von Lymphocyten; und
(3) Binden der aktivierten Lymphocyten an (b) die Antilymphocyt-Antikörperkomponente;
worin die Schritte (1), (2) und (3) gleichzeitig in beliebiger Reihenfolge durchgeführt werden können, mit der Maßgabe, daß Schritt (2) immer gleichzeitig mit oder vor Schritt (3) durchgeführt wird.

6. Verwendung eines bifunktionalen heterodimeren Antikörperkonjugats nach einem der Ansprüche 1 bis 4 zur Herstellung eines pharmazeutischen Mittels, das zur selektiven Zerstörung von Zielzellpopulationen geeeignet ist.

7. Verwendung nach Anspruch 6, worin die Zielzellen **in vitro** zerstört werden.

8. Verwendung nach Anspruch 5, worin die Lymphocyten mit einem Vertreter, ausgewählt aus Antikörpern, Mitogenen, Antigenen und Lymphokinen aktiviert werden.

9. Verwendung nach Anspruch 8, worin die Lymphocyten mit einem oder mehreren Antikörpern aktiviert werden.

10. Verwendung nach Anspruch 9, worin die Lymphocyten mit einem oder mehreren Antikörpern, ausgewählt aus Anti-T11₂, Anti-T3 und Anti-2H1 aktiviert werden.

11. Verwendung nach Anspruch 8, worin die Lymphocyten mit einer Mischung von Anti-T11₂- und Anti-T11₃-Antikörpern aktiviert werden.

12. Verwendung nach Anspruch 8, worin die AntiLymphocyt-Antikörperkomponente (b) des bifunktionalen heterodimeren Antikörperkonjugats ein Anti-T11₃-Antikörper ist, und die Lymphocyten mit Anti-T11₂ aktiviert werden.

## Patentansprüche (Patentansprüche für folgende(n) Vertragsstaat(en): ES, GR)

1. Bifunktionales heterodimeres Antikörperkonjugat, umfassend (a) ein Anti-Zielzellen-Antikörperkomponente, verbunden an (b) eine Antilymphocyt-Antikörperkomponente, worin die Antiiymphocyt-Antikörperkomponente spezifisch für aktivierte Lymphocyten ist, und nicht an normale, nichtaktivierte Lymphocyten bindet.

2. Bifunktionales heterodimeres Antikörperkonjugat nach Anspruch 1, worin die Antilymphocyt-Antikörperkomponente (b) ein Anti-T11₃ - Antikörper ist.

3. Bifunktionales heterodimeres Antikörperkonjugat nach Anspruch 1 oder 2, worin die Antizieizellen-Antikörperkomponente (a) ausgewählt ist aus einem Antitumorzell-Antikörper, einem Antikörper gegen eine Virus-infizierte Zeile, einem Antikörper gegen eine parasitär infizierte Zeile, einem Antikörper gegen eine Autoimmunzelle, einem Antikörper gegen aktivierte Zeilen, die Transplantatabstoßung bewirken, und einem Antikörper gegen aktivierte Zellen, die Transplantat/Wirtskrankheit verursachen.

4. Bifunktionales heterodimeres Antikörperkonjugat nach Anspruch 1, worin die Antikörperkomponenten (a) und (b) des heterodimeren Antikörperkonjugats durch Vernetzung komplimentär reaktiver Gruppen verbunden sind.

5. Verfahren zur Herstellung eines bifunktionalen heterodimeren Antikörperkonjugats, umfassend Verbinden (a) einer Antizieizeiien-Antikörperkomponente an (b) eine Antiiymphocyt-Antikörperkomponente, worin die Antiiymphocyt-Antikörperkomponente spezifisch für aktivierte Lymphocyten ist, und nicht an normale, nichtaktivierte Lymphocyten bindet.

6. Verfahren nach Anspruch 5, worin die Antilymphocyt-Antikörperkomponente (b) ein Anti-T11₃ - Antikörper ist.

7. Verfahren nach Anspruch 5 oder 6, worin die Antizieizeiien-Antikörperkomponente (a) ausgewählt ist aus einem Antitumorzell-Antikörper, einem Antikörper gegen eine Virus-infizierte Zeile, einem Antikörper gegen eine parasitär infizierte Zeile, einem Antikörper gegen eine Autoimmunzelle, einem Antikörper gegen aktivierte Zellen, die Transplantatabstoßung bewirken, und einem Antikörper gegen aktivierte Zeilen, die Transplantat/Wirtskrankheit verursachen.

8. Verfahren nach Anspruch 5, worin die Antikörperkomponenten (a) und (b) des heterodimeren Antikörperkonjugats durch Vernetzung komplimentär reaktiver Gruppen verbunden sind.

9. **In vitro** Verwendung eines heterodimeren Antikörperkonjugats nach einem der Ansprüche 1 bis 4, worin die folgenden Schritte durchgeführt werden:
(1) Binden des bifunktionalen heterodimeren Antikörperkonjugats durch (a) die Antizielzellen-Antikörperkomponente an die Zielzellen;
(2) Aktivieren von Lymphocyten; und
(3) Binden der aktivierten Lymphocyten an (b) die Antilymphocyt-Antikörperkomponente;
worin die Schritte (1), (2) und (3) gleichzeitig in beliebiger Reihenfolge durchgeführt werden können, mit der Maßgabe, daß Schritt (2) immer gleichzeitig mit oder vor Schritt (3) durchgeführt wird.

10. Verwendung eines bifunktionalen heterodimeren Antikörperkonjugats nach einem der Ansprüche 1 bis 4 zur Herstellung eines pharmazeutischen Mittels, das zur selektiven Zerstörung von Zielzellpopulationen geeeignet ist.

11. Verwendung nach Anspruch 10, worin die Zielzellen **in vitro** zerstört werden.

12. Verwendung nach Anspruch 9, worin die Lymphocyten mit einem Mitglied, ausgewählt aus Antikörpern, Mitogenen, Antigenen und Lymphokinen aktiviert werden.

13. Verwendung nach Anspruch 12, worin die Lymphocyten mit einem oder mehreren Antikörpern aktiviert werden.

14. Verwendung nach Anspruch 13, worin die Lymphocyten mit einem oder mehreren Antikörpern, ausgewählt aus Anti-T11₂, Anti-T3 und Anti-2H1 aktiviert werden.

15. Verwendung nach Anspruch 12, worin die Lymphocyten mit einer Mischung von Anti-T11₂- und Anti-T11₃-Antikörpern aktiviert werden.

16. Verwendung nach Anspruch 12, worin die AntiLymphocyt-Antikörperkomponente (b) des bifunktionalen heterodimeren Antikörperkonjugats ein Anti-T11₃-Antikörper ist, und die Lymphocyten mit Anti-T11₂ aktiviert werden.

17. Verfahren zur in vitro Verwendung eines bifunktionalen heterodimeren Antikörperkonjugats nach einem der Ansprüche 1 bis 4 oder wie hergestellt nach einem der Ansprüche 5 bis 8, worin die folgenden Schritte ausgeführt werden:
(1) Binden des bifunktionalen heterodimeren Antikörperkonjugats durch (a) die Antizielzellen-Antikörperkomponente an die Zielzellen;
(2) Aktivieren von Lymphocyten; und
(3) Binden der aktivierten Lymphocyten an (b) die Antilymphocyt-Antikörperkomponente;
worin die Schritte (1), (2) und (3) gleichzeitig in beliebiger Reihenfolge durchgeführt werden können, mit der Maßgabe, daß Schritt (2) immer gleichzeitig mit oder vor Schritt (3) durchgeführt wird.

18. Verfahren zur Herstellung eines pharmazeutischen Mittels, das zur selektiven Zerstörung von Zielzellpopulationen geeignet ist, durch Formulieren eines bifunktionalen heterodimeren Antikörperkonjugats nach einem der Ansprüche 1 bis 4, oder hergestellt nach einem der Ansprüche 5 bis 8, zusammen mit einem pharmazeutisch annehmbaren Träger oder Verdünner.

19. Verfahren nach Anspruch 17, worin die Lymphocyten aktiviert werden mit einem Vertreter, ausgewählt aus Antikörpern, Mitogenen, Antigenen und Lymphokinen.

20. Verfahren nach Anspruch 19, worin die Lymphocyten mit einem oder mehreren Antikörpern aktiviert werden.

21. Verfahren nach Anspruch 20, worin die Lymphocyten mit einem oder mehreren Antikörpern, ausgewählt aus Anti-T11₂, Anti-T3 und Anti-2H1 aktiviert werden.

22. Verfahren nach Anspruch 19, worin die Lymphocyten mit einer Mischung von Anti-T11₂- und Anti-T11₃-Antikörpern aktiviert werden.

23. Verfahren nach Anspruch 19, worin die Antilymphocyt-Antikörperkomponente (b) des bifunktionalen heterodimeren Antikörperkonjugats ein Anti-T11₃-Antikörper ist, und die Lymphocyten mit Anti-T11₂ aktiviert werden.

## Revendications (Revendications pour l'(les) Etat(s) contractant(s) suivant(s): AT, BE, CH, DE, FR, GB, IT, LI, LU, NL, SE)

1. Conjugué d'anticorps hétérodimère bifonctionnel comprenant (a) un composant anticorps anti-cellules cibles réuni à (b) un composant anticorps antilymphocytaire, dans lequel le composant anticorps antilymphocytaire est spécifique des lymphocytes activés et ne se fixe pas à des lymphocytes non activés normaux.

2. Conjugué d'anticorps hétérodimère bifonctionnel selon la revendication 1, dans lequel ledit composant anticorps antilymphocytaire (b) est un anticorps anti-Tll₃.

3. Conjugué d'anticorps hétérodimère bifonctionnel selon la revendication 1 ou 2, dans lequel ledit composant anticorps anti-cellules cibles (a) est choisi dans le groupe constitué par les anticorps anti-cellules tumorales, les anticorps contre les cellules infectées par un virus, les anticorps contre les cellules infectées par un parasite, les anticorps contre les cellules autoimmunes, les anticorps contre les cellules activées provoquant le rejet d'un greffon et les anticorps contre les cellules activées provoquant des réactions du greffon contre l'hôte.

4. Conjugué d'anticorps hétérodimère bifonctionnel selon la revendication 1, dans lequel lesdits composants anticorps (a) et (b) du conjugué d'anticorps hétérodimère sont réunis par des groupes réactifs complémentaires réticulés.

5. Utilisation in-vitro d'un conjugué d'anticorps hétérodimère bifonctionnel selon l'une quelconque des revendications 1 à 4, dans laquelle on effectue les étapes suivantes :
(1) la fixation, aux cellules cibles, du conjugué d'anticorps hétérodimère bifonctionnel, grâce a (a) le composant anticorps anti-cellules cibles ;
(2) l'activation des lymphocytes ; et
(3) la fixation des lymphocytes activés à (b) le composant anticorps antilymphocytaire ;
dans laquelle lesdites étapes (1), (2) et (3) peuvent être mises en oeuvre simultanément ou dans un ordre quelconque, du moment que l'étape (2) est toujours conduite en même temps que l'étape (3) ou avant elle.

6. Utilisation d'un conjugué d'anticorps hétérodimère bifonctionnel selon l'une quelconque des revendications 1 à 4 pour la préparation d'un agent pharmaceutique adapté à la destruction sélective des populations de cellules cibles.

7. Utilisation selon la revendication 6, dans laquelle les cellules cibles sont détruites in vitro.

8. Utilisation selon lit revendication 5, dans laquelle lesdits lymphocytes sont activés par un élément choisi dans le groupe constitué par les anticorps, les mitogènes, les antigènes et les lymphokines.

9. Utilisation selon la revendication 8, dans laquelle les lymphocytes sont activés par un ou plusieurs anticorps.

10. Utilisation selon lit revendication 9, dans laquelle les lymphocytes sont activés par un ou plusieurs anticorps choisi dans le groupe constitué par les anticorps anti-Tll₂, anti-T3 et anti-2H1.

11. Utilisation selon la revendication 8, dans laquelle les lymphocytes sont activés par un mélange d'anticorps anti-Tll₂ et anti-Tll₃.

12. Utilisation selon la revendication 8, dans laquelle ledit composant anticorps antilymphocytaire (b) dudit conjugué d'anticorps hétérodimère bifonctionnel est un anticorps anti-Tll₃ et lesdits lymphocytes sont activés par l'anticorps anti-Tll₂.

## Revendications (Revendications pour l'(les) Etat(s) contractant(s) suivant(s): ES, GR)

1. Conjugué d'anticorps hétérodimère bifonctionnel comprenant (a) un composant anticorps anti-cellules cibles réuni à (b) un composant anticorps antilymphocytaire, dans lequel le composant anticorps antilymphocytaire est spécifique des lymphocytes activés et ne se fixe pas à des lymphocytes non activés normaux.

2. Conjugué d'anticorps hétérodimère bifonctionnel selon la revendication 1, dans lequel ledit composant anticorps antilymphocytaire (b) est un anticorps anti-Tll₃.

3. Conjugué d'anticorps hétérodimère bifonctionnel selon la revendication 1 ou 2, dans lequel ledit composant anticorps anti-cellules cibles (a) est choisi dans le groupe constitué par les anticorps anti-cellules tumorales, les anticorps contre les cellules infectées par un virus, les anticorps contre les cellules infectées par un parasite, les anticorps contre les cellules autoimmunes, les anticorps contre les cellules activées provoquant le rejet d'un greffon et les anticorps contre les cellules activées provoquant des réactions du greffon contre l'hôte.

4. Conjugué d'anticorps hétérodimère bifonctionnel selon la revendication 1, dans lequel lesdits composants anticorps (a) et (b) du conjugué d'anticorps hétérodimère sont réunis par des groupes réactifs complémentaires réticulés.

5. Procédé de préparation d'un conjugué d'anticorps hétérodimère bifonctionnel, comprenant le fait de réunir (a) un composant anticorps anti-cellules cibles à (b) un composant anticorps antilymphocytaire, dans lequel le composant anticorps antilymphocytaire est spécifique des lymphocytes activés et ne se fixe pas à des lymphocytes non-activés normaux.

6. Procédé selon la revendication 5, dans lequel ledit composant anticorps antilymphocytaire (b) est un anticorps anti-Tll₃.

7. Procédé selon lit revendication 5 ou 6, dans lequel ledit composant anticorps anti-cellules cibles (a) est choisi dans le groupe constitué par les anticorps anti-cellules tumorales, les anticorps contre les cellules infectées par un virus, les anticorps contre les cellules infectées par un parasite, les anticorps contre les cellules autoimmunes, les anticorps contre les cellules activées provoquant le rejet d'un greffon et les anticorps contre les cellules activées provoquant des réactions du greffon contre l'hôte.

8. Procédé selon la revendication 5, dans lequel lesdits composants anticorps (a) et (b) du conjugué d'anticorps hétérodimère sont réunis par des groupes réactifs complémentaires réticulés.

9. Utilisation in-vitro d'un conjugué d'anticorps hétérodimère bifonctionnel selon l'une quelconque des revendications 1 à 4 ou préparé selon l'une quelconque des revendications 5 à 8, dans laquelle on effectue les étapes suivantes :
(1) la fixation, aux cellules cibles, du conjugué d'anticorps hétérodimère bifonctionnel, grâce à (a) le composant anticorps anti-cellules cibles ;
(2) l'activation des lymphocytes ; et
(3) la fixation des lymphocytes activés à (b) le composant anticorps antilymphocytaire ;
dans laquelle lesdites étapes (1), (2) et (3) peuvent être mises en oeuvre simultanément ou dans un ordre quelconque, du moment que l'étape (2) est toujours conduite en même temps que l'étape (3) ou avant elle.

10. Utilisation d'un conjugué d'anticorps hétérodimère bifonctionnel selon l'une quelconque des revendications 1 à 4 ou préparé selon l'une quelconque des revendications 5 à 8, pour la préparation d'un agent pharmaceutique adapté à la destruction sélective des populations de cellules cibles.

11. Utilisation selon la revendication 10, dans laquelle les cellules cibles sont détruites in vitro.

12. Utilisation selon la revendication 9, dans laquelle lesdits lymphocytes sont activés par un élément choisi dans le groupe constitué par les anticorps, les mitogènes, les antigènes et les lymphokines.

13. Utilisation selon la revendication 12, dans laquelle les lymphocytes sont activés par un ou plusieurs anticorps.

14. Utilisation selon la revendication 13, dans laquelle les lymphocytes sont activés par un ou plusieurs anticorps choisi dans le groupe constitué par les anticorps anti-Tll₂, anti-T3 et anti-2H1.

15. Utilisation selon la revendication 12, dans laquelle les lymphocytes sont activés par un mélange d'anticorps anti-Tll₂ et anti-Tll₃.

16. Utilisation selon la revendication 12, dans laquelle ledit composant anticorps antilymphocytaire (b) dudit conjugué d'anticorps hétérodimère bifonctionnel est un anticorps anti-Tll₃ et lesdits lymphocytes sont activés par l'anticorps anti-Tll₂.

17. Procédé d'utilisation in vitro d'un conjugué d'anticorps hétérodimère bifonctionnel selon l'une quelconque des revendications 1 à 4 ou préparé selon l'une quelconque des revendications 5 à 8, dans lequel on effectue les étapes suivantes :
(1) la fixation, aux cellules cibles, du conjugué d'anticorps hétérodimère bifonctionnel, grâce à (a) le composant anticorps anti-cellules cibles ;
(2) l'activation des lymphocytes ; et
(3) la fixation des lymphocytes activés à (b) le composant anticorps antilymphocytaire ;
dans laquelle lesdites étapes (1), (2) et (3) peuvent être mises en oeuvre simultanément ou dans un ordre quelconque, du moment que l'étape (2) est toujours conduite en même temps que l'étape (3) ou avant elle.

18. Procédé de préparation d'un agent pharmaceutique adapté à la destruction sélective de populations de cellules cibles, consistant à formuler un conjugué d'anticorps hétérodimère bifonctionnel selon l'une quelconque des revendications 1 à 4 ou préparé selon l'une quelconque des revendications 5 à 8, avec un vecteur ou diluant acceptable en pharmacie.

19. Procédé selon la revendication 17, dans lequel lesdits lymphocytes sont activés par un élément choisi dans le groupe constitué par les anticorps, les mitogènes, les antigènes et les lymphokines.

20. Procédé selon la revendication 19, dans lequel les lymphocytes sont activés par un ou plusieurs anticorps.

21. Procédé selon la revendication 20, dans lequel les lymphocytes sont activés par un ou plusieurs anticorps choisis dans le groupe constitué par les anticorps anti-Tll₂, anti-T3 et anti-2H1.

22. Procédé selon la revendication 19, dans lequel les lymphocytes sont activés par un mélange d'anticorps anti-Tll₂ et anti-Tll₃.

23. Procédé selon la revendication 19, dans lequel ledit composant anticorps antilymphocytaire (b) dudit conjugué d'anticorps hétérodimère bifonctionnel est un anticorps anti-Tll₃ et lesdits lymphocytes sont activés par l'anticorps anti-Tll₂.
